(19) Europäisches Patentamt
European Patent Office
Office européen des brevets

(11) EP 1 402 886 B1

(12) EUROPEAN PATENT SPECIFICATION

(45) Date of publication and mention of the grant of the patent:
**14.02.2007 Bulletin 2007/07**

(21) Application number: **02730716.4**

(22) Date of filing: **24.05.2002**

(51) Int Cl.:
***A61K 31/05*** *(2006.01)* ***A61K 31/12*** *(2006.01)*
***A61K 31/7028*** *(2006.01)* ***A61K 8/00*** *(2006.01)*
***A61Q 1/00*** *(2006.01)* ***A61P 3/04*** *(2006.01)*
***A61P 3/06*** *(2006.01)* ***A61P 1/16*** *(2006.01)*
***A23L 1/30*** *(2006.01)*

(86) International application number:
**PCT/JP2002/005090**

(87) International publication number:
**WO 2002/096399 (05.12.2002 Gazette 2002/49)**

(54) **LIPOLYSIS ACCELERATOR FOR REDUCING BODY WEIGHT AND MEDICINAL USE**

LIPOLYSE-BESCHLEUNIGER ZUR REDUZIERUNG DES KÖRPERGEWICHTS UND MEDIZINISCHE ANWENDUNG

ACCELERATEUR DE LIPOLYSE POUR REDUIRE LA MASSE CORPORELLE ET POUR UTILISATION MEDICALE

(84) Designated Contracting States:
**DE FR GB**

(30) Priority: **28.05.2001 JP 2001158268**
**04.03.2002 JP 2002057996**

(43) Date of publication of application:
**31.03.2004 Bulletin 2004/14**

(73) Proprietor: **Kao Corporation**
**Chuo-ku**
**Tokyo 103-8210 (JP)**

(72) Inventors:
- **HARA, Mariko**
  **San Francisco, CA 94122 (US)**
- **SATO, Yurie**
  **Odawara-shi, Kanagawa 250-0002 (JP)**
- **IKEMOTO, Takeshi**
  **Minamiashigara-shi, Kanagawa 250-0113 (JP)**
- **INOUE, Shintaro**
  **Odawara-shi, Kanagawa 250-0851 (JP)**
- **NISHIO, Hiroyuki**
  **Fujisawa-shi, Kanagawa 251-0023 (JP)**
- **MORIMOTO, Yasuo**
  **Osaka-shi, Osaka 534-0016 (JP)**

(74) Representative: **Prins, Adrianus Willem et al**
**Vereenigde,**
**P.O.Box 87930**
**2508 DH Den Haag (NL)**

(56) References cited:
**EP-A- 0 930 019** **FR-A- 2 716 374**
**JP-A- 2000 169 325** **JP-A- 2000 239 143**
**US-A- 5 629 338** **US-A- 5 674 498**
**US-A1- 2002 042 441**

- **PATENT ABSTRACTS OF JAPAN vol. 1998, no. 11, 30 September 1998 (1998-09-30) & JP 10 158181 A (KANEBO LTD), 16 June 1998 (1998-06-16)**
- **DATABASE WPI Week 199823, Derwent Publications Ltd., London, GB; Class B04, AN 1998-259226 & RU 2 089 213 A (GRADIENT ENTERP) 10 September 1997**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 402 886 B1

## Description

FIELD OF THE INVENTION

The present invention relates to a lipolysis promoter which is useful to improve obese constitution by promoting shrinkage of general or topical fatty tissues, or to suppress or prevent obesity by preventing the fatty tissue from growing. The present invention relates also to a cosmetic composition, a food or drink composition each comprising the same, reducing body weight by a lipolitic function thereof and a drug comprising the same.

DESCRIPTION OF THE PRIOR ART

Recently, with increase in total calorie intake, especially increase in fat intake, more and more people suffer from obesity. Obesity is a state of a body in which excessive intercorporal fat is accumulated due to over-nourishment. The intercorporal fat is produced from energy intake exceeding energy consumption and accumulates as a neutral fat in white adipose tissues. Obesity due to heavily accumulated intercorporal fat is not only aesthetically unfavorable, but also causes various diseases, such hyperlipidemia, i.e., hypercholesterolemia or hypertriglyceridemia, fatty liver, diabetes, and high blood pressure.

Among the diseases, hyperlipidemia is a state of body in which one or more of the components of serum lipids such as cholesterol, triglyceride, phospholipid, and free fatty acids increase abnormally. If this state lasts long, cholesterol tends to deposit on arterial conduits to cause arterioschlerosis.

Fatty liver is a state of a body in which lipids, particularly neutral fat, accumulate excessively in a liver due to over-nutrition and/or alcohol intake. Especially, fatty liver of an obese body due to over-nutrition is known to be caused by increase in mobilization of fatty acids from peripheral tissues due to excessive calorie intake, and by accelerated synthesis of fatty acid and neutral fat in a liver (Sogo Rinsho, Vol 50, No.12, pp3215-3221, 2001).

There are some therapies for hyperlipidemia or fatty liver such as diet therapy and exercise therapy. If the symptoms are not relieved by these therapies, drugs are used. Drugs which are being currently used include HMG-CoA reductase inhibitors and Fibrates drugs (Journal of the Medical Society of Showa, Vol.58, No.5, pp397-401, 1998).

Meanwhile, people, particularly women, yearn for a slim and tight body from the viewpoint of appearance. However, accumulation of intracelial viscera fat such as mesentery fat and omental fat often takes place not only in obese people but also in those with normal weight(Sogo Rinsho, Vol.50, No.12, pp3277-3282).

For the aforesaid reasons, a lipolysis promoter is desired to cure obesity in appearance due to accumulation of intercorporal fat and to lipolyze intracelial viscera fat as well.

It is difficult to diminish accumulated fat cells, but it is easier to make the cells smaller by decomposing lipid droplets in the cells.

The lipid droplets can be decomposed with an enzyme, phospholipase C, in a similar manner as protein. However, they are surrounded by a hydrophobic phospholipid membrane, so that the enzyme present in endoplasmic reticulum which is hydrophilic medium cannot access the lipid droplets. Meanwhile, sympathetic nerves are activated by taking exercise to secrete fat decomposing hormone. This hormone is believed to interact with the phospholipid membrane to allow the enzyme to obtain access to the lipid droplets to thereby promote lipolysis.

Therefore, the present inventors endeavored to find a substance which promotes accessibility of the enzyme to the lipid droplets in a similar manner as the hormone. It was found that raspberry ketone, zingerone and derivatives thereof promote decomposition of fat accumulated in fatty tissues and, thus, are effective to suppress obesity or to reform obese constitution (Japanese Patent Application Laid-open No. 2000-169325).

Although both raspberry ketone and zingerone are effective as lipolysis promoters, they have peculiar odor and taste. Consequently their amounts of dosage are limited and their versatility of applications as an anti-obesity agent, a therapeutic drug for hyperlipidemia or fatty liver is not satisfactory.

Thus, an object of the present invention is to provide a lipolysis promoter which has a satisfactory versatility with no peculiar odor or taste, an effect of reforming obese constitution by reducing general and topical fat tissues, or an anti-obesity effect by preventing increase of the fat tissues. The present invention also aims to provide a cosmetic composition comprising the same, a food or drink composition comprising the same, reducing body weight based on lipolitic function of the same and a drug comprising the same.

SUMMARY OF THE INVENTION

To achieve the aforesaid object, the present inventors have made researches to find a substance which acts on fat tissues, promote lipolysis, and has less odor or taste. It has been found that a compound represented by the following formula (1) promotes the lipolysis of accumulated fat in fat tissues and has remedial effects of obesity, hyperlipidemia and fatty liver by the lipolitic function:

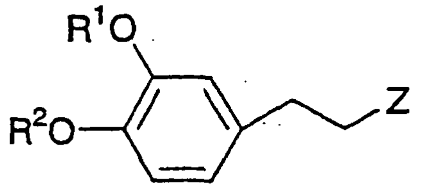

wherein Z is a group represented by the formula (2) or (3);

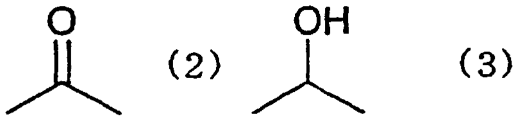

and either of $R^1$ and $R^2$ is a hydrogen atom and the other is a hydrogen atom or a glucose residue.

Thus, the present invention provides a lipolysis promoter comprising the compound represented by the formula (1) as an active ingredient, a cosmetic composition comprising the same, a food or drink composition comprising the same, reducing body weight by the lipolitic function thereof and a drug comprising the same.

BRIEF DESCRIPTION OF THE DRAWINGS

Fig. 1 is a graph which shows amounts of by thus subcutaneous fat of a group of rats fed with a high-fat feed comprising the present lipolysis promoter, a group of rats fed with a high-fat feed, and a group of rats fed with a standard feed.

Fig. 2 is a graph which shows amounts of fat around uterus and retroperitoneum fat of a group of rats fed with a high-fat feed comprising the present lipolysis promoter, a group of rats fed with a high-fat feed, and a group of rats fed with a standard feed.

Fig. 3 is a graph which shows amounts of mesentery fat of a group of rats fed with a high-fat feed comprising the present lipolysis promoter, a group of rats fed with a high-fat feed, and a group of rats fed with a standard feed.

Fig. 4 is a graph showing weekly changes of serum triglyceride amounts, where the circle represents an serum triglyceride amount of a group of rats fed with a high-fat feed comprising the present lipolysis promoter, the triangle that of a group fed with a high-fat feed, and the square represents that of a group fed with a standard feed.

In the figures, a single asterisk means that there was a significant difference with p<0.05 in Dunnett's test from a high-fat-fed group; double asteriks means that there was a significant difference with p<0.01 in Dunnett's test from a high-fat-fed group; and double pounds means that there was a significant difference with p<0.01 in Student's test from a high-fat-fed group

DESCRIPTION OF THE PREFERRED EMBODIMENTS

The present invention will be explained in detail. The compounds used in the present invention are as follows:

4-(3', 4'- dihydroxyphenyl)- butane- 2-one,
4-(3'-glucosyl-4'- hydroxyphenyl)- butane- 2-one,
4-(4'-glucosyl-3'- hydroxyphenyl)- butane- 2-one,
4-(3', 4'- dihydroxyphenyl)- butane- 2-ol,
4-(3'-glucosyl-4'- hydroxyphenyl)- butane- 2-ol, and
4-(4'-glucosyl-3'- hydroxyphenyl)- butane- 2-ol.

Methods for preparing the compounds used in the present invention will be explained. Some of the compounds used in the present invention were proposed to be used in a skin cosmetic compositions having a melanogenesis

inhibition effect by the present inventors in Japanese Patent Application Laid-open No. 2000-239143.

The compound of the aforesaid formula (1) wherein Z is a group represented by the formula (2), and $R^1$ and $R^2$ are both a hydrogen atom, i.e.,4-(3', 4'- dihydroxyphenyl)- butane-2-one, may be easily prepared in the method described in the above patent publication. Specifically, it may be easily prepared by removing methylether from zingerone, 4-(3'-methoxy-4'-hydroxyphenyl)-butane-2-one, which is contained in ginger, in a known manner with, for example, boron tribromide or hydriodic acid.

The compound of the formula (1) wherein $R^1$ or $R^2$ is a glucose residue may be obtained by extracting it from plants, such as raspberries, with an appropriate solvent and, if necessary, condensing or evaporating the solvent to dryness in a known method (see Phytochemistry, Vol. 29, No.12, 3853-3858(1990)).

The compound of the aforesaid formula (1) wherein Z is a group represented by the forumula (3), and $R^1$ and $R^2$ are both a hydrogen atom, 4-(3', 4'- dihydroxyphenyl)- butane-2-ol, may be prepared in a similar manner as described above, from 4-(3'-methoxy-4'-hydroxyphenyl) -butane-2-ol obtained by reduction of zingerone with sodium borohydride.

These compounds may be prepared by extracting natural products. Using 4-(3', 4'- dihydroxyphenyl)- butane-2-one or 4-(3', 4'-dihydroxyphenyl)- butane- 2-ol, corresponding glucosides can be prepared according to the method described in S.Mabic et al., Tetrahedron Letter, Vol.34, No.28, 4531-4534(1993).

The compound of the formula (1) may be used alone or in combination of two or more of them as a lipolysis promoter. The amount of the compound to be incorporated in the food or drink composition or the drug may vary depending on their actual forms. Preferably, in the food or drink composition, the compound is incorporated in an amount of from 0.001 to 20 wt%, more preferably from 0.01 to 10 wt%, most preferably from 0.01 to 3.0 wt%, based on the weight of the composition. If the amount is less than the aforesaid lower limit, the effect of the present invention may not be attained. If the amount is more than the aforesaid upper limit, the effect may not increase correspondingly.

The amount of the present compound to be incorporated in the drug may vary depending on conditions of disease, age and weight of a patient. Preferably, for an adult, the present compound is orally or parenterally administered in an amount of from 1 mg to 5000 mg, more preferably from 50 mg to 2000 mg, in one or several doses per day.

The present lipolysis promoter can be incorporated in food or drink compositions or drugs of any dosage form.

The food or drink compositionmay be chewing gum, chocolate, candies, gummy jellies, beverages, soup, ice cream, noodles, bakery products or alcoholic drinks.

In the present food or drink composition, any known substances can be incorporated in addition to the aforesaid present compounds, for example, those conventionally used in a food or drink composition, such as saccharides, flavors, emulsifiers, milk products, proteins, stabilizers, colorants, acidulants, fats and oils, cereals, eggs, and gum base; xanthine derivative such as caffeine; hydroxy citric acid; and those which suppress or prevent obesity, such as capsaicin, raspberry ketone, and zingerone.

The present invention encompasses reducing body weight by using the food or drink composition.

The present drug comprising the present lipolysis promoter may be in the form of an agent for oral administration such as a capsule, a tablet, a granule, a parvule, powder and a liquid, or an agent for parenteral administration such as injection or external application.

The present drug may be the compound represented by the formula (1) alone. The drug for oral administration may be formulated in any conventional manner by adding, if necessary, conventional drug additives such as excipient and disintegrant, for example, lactose, hydroxypropylmethyl cellulose, hydroxypropylcellulose, low substituted hydroxypropylcellulose, ethylcellulose, corn starch, crystalline cellulose, carmellose calcium, anhydrous silicic acid, synthetic aluminum silicate and/or magnesium stearate.

The injection may be formulated in any conventional manner by adding; if necessary, isotonicity agents such as mannitol, sodium chloride, glucose, sorbitol, glycerol, xylitol, fructose, maltose, and mannose; stabilizers such as sodium sulfite; preservatives such as benzylalcohol, and methyl parahydroxybenzoate; solubilizers; soothing agents; and pH modifiers.

## EXAMPLES

The present invention will be explained in details with reference to the following Test Examples. Efficacy of lipolysis promotion was evaluated in the lipolysis test and in the subcutaneous fat decomposition test described below in the specification. Efficacy of anti-obesity was evaluated in the test for suppression of body weight gain on high-fat-fed mice and in the test for suppression of intercorporal fat increase using high-fat-fed rats. Remedial efficacy for hyperlipidemia was evaluated on high-fat-fed rats, and remedial efficacy of fatty liver was evaluated on high-fat-fed mice. Commercially available raspberry ketone and zingerone were used.

<u>Preparation Example 1 : 4-(3', 4'-dihydroxyphenyl)-butane-2-one</u>

Zingerone, 4-(3'-methoxy-4'-hydroxyphenyl)-butane-2-one, was dissolved in dichloromethane, to which a 1.0

mol/l boron tribromide solution in dichloromethane was then added at -30°C. While stirring, the reaction mixture was gradually heated to room temperature and allowed to react for further 2 hours at the temperature. After adding ice water to the reaction mixture, an aqueous 2 % sodium hydrogen carbonate solution was added. Then, the reaction mixture was extracted with ethyl acetate, and the ethyl acetate layer thus obtained was dried with anhydrous magnesium sulfate. Then, the ethyl acetate layer was vacuum condensed to obtain brown oil, which was then purified by silica gel chromatography, using a mixture of hexane/ethyl acetate = 7/3 as a developing solvent to thereby obtain 4-(3', 4'-dihydroxyphenyl)-butane-2-one to be used in the present invention.

Preparation Example 2 : 4-(3', 4'-dihydroxyphenyl)-butane-2-ol

The procedures in preparation Example 1 were repeated except that use was made of 4-(3'-methoxy-4'-hydroxyphenyl)-butane-2-olobtained by reduction of zingerone with sodium borohydride, instead of zingerone.

Test Example 1: Determination of Lypolytic Activity

(1) Test Method

Free fat cells were prepared from epididymis fatty tissues of Wister male rats (body weight, 150 to 200g) using a collagenase solution, according to Rodbell's method(M. Rodbell, J.Biol.Chem., 239,375(1964)). The cells were added to a Hanks' balanced salt solution containing 0.05 μg/ml of bovine serum albumin, 0.05 μg/ml of norepinephrine and 100μg/ml of the present compound to be tested, and then allowed to react at 37°C for 1 hour. Liberated fatty acids were extracted and quantified with a copper reagent and a color developing reagent. Lypolytic activity was calculated according to the following equation.

$$\text{Lypolytic activity } (\%) = [A/B] \times 100,$$

wherein

A: amount of fatty acids in a sample solution, and
B: that in a control solution without present compound.

(2) Test Results

The results are shown in Table 1.

Table 1

| Substance tested | Degree of the lipolysis promotion (%) |
|---|---|
| 4-(3', 4'- dihydroxyphenyl)- butane-2-one, the present lipolysis promoter | 194.3 +/- 12.4 p< 0.001 |
| Raspberry ketone, the compound used in Comparative Example described below | 142.0 +/- 6.8 p<0.01 |

As is clear from Table 1, the present lipolysis promoter, 4-(3', 4'-dihydroxyphenyl)-butane-2-one, significantly promoted lipolysis compared to Comparative Example.

Test Example 2:Subcutaneous Fat Lipolysis Test

(1) Test Method

Wister rats (male, 7 to 9 weeks aged) were shaved at their bellies. Then, the belly cortex was enucleated together with subcutaneous fatty tissues and mounted on Franz-type diffusion cells with a diameter of 2 cm. The keratin held in the upper cell was uniformly coated with 0.5 g of the compositions of Examples 1,2, Comparative Example and Referential Example described below, and the lower cell for the subcutaneous tissue side was filled with a phosphate-buffered physiological saline at pH 7.2. After keeping the cells at 37°C for 6 hours, an aliquot of the buffered solution was taken out from the lower cell and glycerol liberated in the solution was quantified by an enzyme method with F-kit Glycerol, ex Beringer Manheim Co. For each sample, the quantification was repeated five times and data were averaged.

(2) Test Results

The test results are as seen in Table 2.

Table 2

| | Liberated Glycerol, μmol/mL |
|---|---|
| Example 1 (comprising 1% of the lipolysis promoter of the present invention) | 174.5 |
| Example 2 (comprising 3% of the lipolysis promoter of the present invention) | 191.6 |
| Comparative Example 2a | 60.8 |
| Referential Example (comprising raspberry ketone) | 132.4 |

As is clear from Table 2, by applying the compositions comprising the present lipolysis promoter (Examples 1 and 2 described below), the amount of liberated glycerol that is a product of the lipolysis, was significantly increased, compared to the composition with no lipolysis promoter (Comparative Example described below). The composition comprising raspberry ketone (Referential Example described below) showed the higher amount of the liberated glycerol, but the compositions comprising the present lipolysis promoter (Examples 1 and 2 described below) showed the still higher amount of the liberated glycerol.

Test Example 3: Body Weight Gain Suppression Test on High-Fat-Fed Mice

(1) Test Method

Three groups of ICR mice (male, 5-week aged, average body weight of 31 g), each consisting of 7 mice, were used. The first group of the mice were fed with a standard feed (solid standard feed MF, ex Oriental Yeast Co.); the second group with the high-fat feed comprising 4-(3', 4'- dihydroxyphenyl)- butane-2-one as shown in Table 3; and the third group with the high-fat feed as shown in Table 3, each for 3 weeks. The mice were weighed when the test started and 3 weeks later. The mice were allowed to freely intake the feed and water.

Table 3

| Component | High-fat feed comprising the present lipolysis promoter | High-fat feed |
|---|---|---|
| Beef tallow | 40 parts by weight | 40 parts by weight |
| Corn starch | 10 parts by weight | 10 parts by weight |
| Granulated sugar | 9 parts by weight | 9 parts by weight |
| Mineral*1 | 4 parts by weight | 4 parts by weight |
| Mineral*2 | 1 part by weight | 1 part by weight |
| Casein | 35 parts by weight | 36 parts by weight |
| 4-(3', 4'-dihydroxyphenyl)-butane-2-one | 1 part by weight | |
| *1 : AIN Mineral Mix, ex ICN Co.<br>*2 : AIN Vitamin Mix, ex ICN Co. | | |

(2) Statistical Analyses

Body weight gains were compared between the group fed with the high-fat feed and the one fed with the high-fat feed with the present lipolysis promoter. Since a significant difference between the groups was seen in one-factor analysis of variance(one way-ANOVA) test, multiple comparisons(post-hoc test) were made.

(3) Test Results

The test results are as seen in Table 4.

Table 4

| Group | Average body weight, g | Body weight gain, g |
|---|---|---|
| High-fat feed with the present lipolysis promoter | 38.4 | 7.4* (*p<0.05:compared with the body weight gain of the high-fat fed group) |
| High-fat feed | 43.2 | 12.2 |
| Standard feed | 38.1 | 7.1 |

As is clear from Table 4, the body weight gain of the group fed with the high-fat feed comprising the present lipolysis promoter was significantly suppressed, compared to the group fed with the high-fat feed without the lipolysis promoter.

Test Example 4: Body Weight Gain Suppression Test on High-Fat-Fed rat

(1) Test Method

Three groups of Sprague-Dawley rats (female, 9-week aged), each consisting of 8 rats, were used. The first group of rats were fed with a standard feed (CE-2, ex Japan Clea Co.) ; the second group with the high-fat feed comprising 4-(3', 4'- dihydroxyphenyl)-butane-2-one; and the third group with the high-fat feed as shown in Table 5, respectively, for 6 weeks. The rats were allowed to freely intake feed. Then, the rats were subjected to an autopsy to extract subcutaneous fat surrounding lower abdomen, fat around uterus, retroperitoneum fat, and mesentery fat and each fat was weighed.

Table 5

| Component | High-fat feed comprising the present lipolysis promoter | High-fat feed |
|---|---|---|
| Beef tallow | 20 parts by weight | 20 parts by weight |
| Cellulose powder | 5 parts by weight | 5 parts by weight |
| α-Starch | 1 part by weight | 1 part by weight |
| Granulated sugar | 10 parts by weight | 10 parts by weight |
| Mineral | 7 parts by weight | 7 parts by weight |
| Vitamin | 1 part by weight | 1 part by weight |
| Casein | 55 parts by weight | 56 parts by weight |
| 4-(3', 4'-dihydroxyphenyl)-butane-2-one | 1 part by weight | |

(2) Statistical Analyses

The amounts of the subcutaneous fat surrounding lower abdomen, fat around uterus, retroperitoneum fat and mesentery fat were compared between the group fed with the high-fat feed and the one fed with the high-fat feed comprising the present lipolysis promoter. Significant difference test was made according to the Dunnett test.

(3) Test Results

The test results are as seen in Figures 1 to 3.

As is clear from Figures 1 to 3, the weights of subcutaneous fat surrounding lower abdomen, fat around uterus, retroperitoneum fat and mesentery fat of the group fed with the high-fat feed comprising the present lipolysis promoter were significantly lower than those of the group fed with the high-fat feed without the lipolysis promoter.

Test Example 5: Test for Evaluating Remedial Efficacy for Hyperlipemia on High-Fat-Fed Mice

(1) Test Method

The same method as in Test Example 4 was used. At the start of the test and every one week after the start, blood was sampled and the amount of serum triglyceride was determined. The rats were allowed to freely intake feed and water.

(2) Statistical Analyses

The amounts of serum triglyceride were compared between the group fed with the high-fat feed and the one fed with the high-fat feed comprising the present lipolysis promoter. Significant difference test was made according to Dunnett test.

(3) Test Results

The results are as seen in Fig. 4.

As is clear from Figure 4, an increase of serum triglyceride was significantly suppressed in the group fed with the high-fat feed comprising the present lipolysis promoter compared to the group fed with the high-fat feed without the lipolysis promoter.

Test Example 6: Test for Evaluating Remedial Efficacy for Fatty Liver on High-Fat-Fed Mice

(1) Test Method

Three groups of ICR mice (male, 5-week aged), each group consisting of 6 mice, were used. Two groups of mice were fed with the high-fat feed shown in Table 3 for 6 weeks. Then, one of the two groups was fed with the high-fat feed comprising 4-(3', 4'- dihydroxyphenyl)-butane-2-one and the other was fed with the high-fat feed shown in Table 3, each for 5 weeks. The third group was fed with a standard feed (MF, ex Oriental Yeast Co.) for 11 weeks. The mice were allowed to freely intake feed and water. On the last day of the test, the mice were fasted for 18 hours. Then, the mice were subjected to an autopsy. After a left part of a liver was perfused with a physiological salt solution, a portion of the perfused part was taken out. One hundred mgs of the liver taken out was homogenized in 0. 9 mL of a physiological salt solution into a 10% homogenized solution. To 0.1 mL of the 10% homogenized solution, 2.4 mL of a mixture of chloroform and methanol with a volume to volume ratio of 2 to 1 was added and mixed by shaking for 2 minutes, and then centrifuged at 800 x g for 10 minutes. Zero point one mL of the chloroform layer was sampled into a test tube. Then, the chloroform in the test tube was evaporated to dryness using Dry Block Bath, ex Iuchi Seieido Ltd., at 70 degrees C under nitrogen gas flow. The amount of triglyceride was calculated from an absorbance datum obtained by using an analysis kit of an enzyme method, ex Wako Pure Chemical Ltd.

(2) Statistical Analyses

The amounts of triglyceride in livers were compared between the group fed with the high-fat feed and the group fed with the high-fat feed comprising the present lipolysis promoter. Since a significant difference between the groups was seen in one-factor ANOVA test, multiple comparisons were made.

(3) Test Results

The results are as seen in Table 6.

Table 6

| Group | Triglyceride in liver, g |
|---|---|
| High-fat feed with the present lipolysis promoter | 16.0** (**$p<0.01$:compared to the toriglyceride of the high-fat fed group) |
| High-fat feed | 35.6 |
| Standard feed | 10.7 |

As is clear from Table 6, an increase of liver triglyceride was significantly suppressed in the group fed with the high-fat feed comprising the present lipolysis promote, compared to the group fed with the high-fat feed without the lipolysis promoter.

The present invention will be explained further with reference to the following Examples, Comparative Example and Referential Example. Comparative examples 1, 2 and 2a, and Referential Example (Gel Type Skin Cosmetic Composition for Reducing Weight)

Parts A and B were separately prepared by mixing and dissolving the substances in the amounts as shown in Table 7 in a conventional method. Then Part B was added to Part A with stirring to obtain a gel type skin cosmetic composition for reducing weight.

Table 7

| Part(wt%) | Example 1 | Example 2 | Comparative Example 2a | Referential Example |
|---|---|---|---|---|
| Part A | | | | |
| 4-(3', 4'- dihydroxy phenyl)-butane-2-one | 1.0 | 3.0 | - | - |
| Raspberry ketone | - | - | - | 1.0 |
| Glycerol | 10.0 | 10.0 | 10.0 | 10.0 |
| Carboxyvinyl polymer | 0.3 | 0.3 | 0.3 | 0.3 |
| Disodium edetate | 0.1 | 0.1 | 0.1 | 0.1 |
| Purified water | balance | balance | balance | balance |
| Diisopropanolamine | 1.0 | 1.0 | 1.0 | 1.0 |
| Squalane | 10.0 | 10.0 | 10.0 | 10.0 |
| Part B | | | | |
| Polyoxyethylene(60) hydrogenated castor oil | 0.8 | 0.8 | 0.8 | 0.8 |
| Carrageenan | 3.0 | 3.0 | 3.0 | 3.0 |
| Xanthan gum | 3.0 | 3.0 | 3.0 | 3.0 |
| Polyvinylalcohol | 2.0 | 2.0 | 2.0 | 2.0 |
| Ethanol | 45.0 | 45.0 | 45.0 | 45.0 |
| 1-Menthol | 0.1 | 0.1 | 0.1 | 0.1 |
| Perfume | q.s. | q.s. | q.s. | q.s. |

Example 3(Chewing Gum)

Chewing gum with the following composition was prepared.

| | wt% |
|---|---|
| Gum base | 20 |
| Maltitol | 73.5 |
| Reduced glutinous starch syrup | 4 |
| Apple flavor | 0.5 |
| Garcinia Cambogia Extract*1 | 1 |
| 4-(3', 4'- dihydroxyphenyl)- butane-2-one | 1 |

*1 : Garcinia Cambogia Extract, Citrimax HCA-500-F(trade name), ex Interhealth Inc., containing 47.5% of hydroxycitic acid(HCA).

Example 4 (Sweet Tablets)

Sweet tablets with the following composition was prepared.

| | wt% |
|---|---|
| Sorbitol | 72.9 |
| Sucrose ester of fatty acid | 4 |
| Raspberry flavor | 0.1 |
| Garcinia Cambogia Extract | 3 |
| 4-(3', 4'- dihydroxyphenyl)- butane-2-one | 10 |

Example 5 (Tablet)

Formulation:

| | parts by weight |
|---|---|
| 4-(3', 4'- dihydroxyphenyl)- butane-2-one | 1 |
| Lactose | 93 |
| Synthetic aluminum silicate | 5 |
| Magnesium stearate | 1 |

Procedures:

The components described above were mixed. The mixture was molded into 300-mg tablets, each containing 30 mg of 4-(3', 4'-dihydroxyphenyl)- butane-2-one.

Example 6 (Tablet)

The procedures of Example 5 were repeated except that 4-(3', 4'-dihydroxyphenyl)- butane-2-ol was used instead of 4-(3', 4'-dihydroxyphenyl)- butane-2-one.

Example 7 (Tablet)

The procedures of Example 5 were repeated except that 4- (3'-glucosyl-hydroxyphenyl)- butane-2-ol was used instead of 4-(3', 4'-dihydroxyphenyl)- butane-2-one.

Example 8 (Skin lotion)

Formulation:

| | Amount, wt% |
|---|---|
| 4-(3', 4'- dihydroxyphenyl)- butane-2-one | 3.0 |
| Ethanol | 10.0 |
| Polyoxyethylene(20) sorbitan monolaurate | 5.0 |
| Dibutylhydroxytoluene | 0.01 |
| Perfume | 0.05 |
| Glycerol | 5.0 |
| Xanthan gum | 0.1 |
| Hydroxyethylcellulose | 0.1 |
| Purified water | balance |

Procedures:

In a mixture of the components from glycerol to purified water in the above list, 4-(3', 4'- dihydroxyphenyl)-butane-2-one was homogeneously dissolved. Then, the mixture obtained and the components from ethanol to perfume were mixed by stirring into a homogeneous dispersion to prepare a skin lotion.

Example 9 (Skin lotion)

The procedures of Example 8 were repeated except that the amount of 4-(3', 4'- dihydroxyphenyl)- butane-2-one was 0.5 wt%.

Example 10 (Skin lotion)

The procedures of Example 8 were repeated except that the amount of 4-(3', 4'- dihydroxyphenyl)- butane-2-one was 0.01 wt%.

Example 11 (Skin lotion)

The procedures of Example 8 were repeated except that 4-(3', 4'-dihydroxyphenyl)- butane-2-ol was used instead of 4-(3', 4'-dihydroxyphenyl)- butane-2-one.

Example 12 (Skin lotion)

The procedures of Example 11 were repeated except that the amount of 4-(3', 4'- dihydroxyphenyl)- butane-2-ol was 0.5 wt%.

Example 13 (Skin lotion)

The procedures of Example 11 were repeated except that the amount of 4-(3', 4'- dihydroxyphenyl)- butane-2-ol was 0.01 wt%.

Example 14 (Skin cream)

Formulation

| | Amount, wt% |
|---|---|
| 4-(3', 4'- dihydroxyphenyl)- butane-2-one | 3.0 |
| Glycerol monostearate | 2.0 |
| Beeswax | 1.0 |
| Polyoxyethylene(6) sorbitan monolaurate | 1.0 |
| Vaseline | 4.0 |
| Liquid paraffin | 12.0 |
| Sodium N-stearoyl-L-glutamate | 5.0 |
| Carrageenan | 0.3 |
| Methylparaben | 0.1 |
| Purified water | balance |

Procedures

A mixture of 4- (3', 4'-dihydroxyphenyl) - butane-2-one and the above components from sodium N-stearoyl-L-glutamate to purified water, and a mixture of the components from glycerol monostearate to liquid paraffin were separately heated to 80°C to make homogeneous solutions. The former mixture was poured into the latter mixture and emulsified. Then, the emulsion was cooled with stirring to thereby obtain skin cream.

Example 15 (Skin cream)

The procedures of Example 14 were repeated except that the amount of 4-(3', 4'- dihydroxyphenyl)- butane-2-one was 0.5 wt%.

Example 16 (Skin cream)

The procedures of Example 14 were repeated except that the amount of 4-(3', 4'- dihydroxyphenyl)- butane-2-one was 0.01 wt%.

Example 17 (Skin cream)

The procedures of Example 14 were repeated except that 4-(3', 4'-dihydroxyphenyl)- butane-2-ol was used instead of 4-(3', 4'-dihydroxyphenyl)- butane-2-one.

Example 18 (Skin cream)

The procedures of Example 17 were repeated except that the amount of 4-(3', 4'- dihydroxyphenyl)- butane-2-ol was 0.5 wt%.

Example 19 (Skin cream)

The procedures of Example 17 were repeated except that the amount of 4-(3', 4'- dihydroxyphenyl)-butane-2-ol was 0.01 wt%.

All of the skin cosmetics, food, and drugs of Examples 1 to 19 showed the advantages of the present invention. However, examples 1, 2, 2a and 18-19 are to be considered as merely illustrative and not falling within the scope of the invention as claimed.

INDUSTRIAL APPLICABILITY

As shown in the aforesaid Test Examples and Examples, the compound represented by the formula (1) promotes lipolysis, suppresses weight gain and decreases triglyceride in serum and a liver by its lipolitic function. It does not have any peculiar odor or taste, and thus has has versatility. It is a very useful lipolysis promoter. Therefore, the present invention provides the lipolysis promoter which is useful to reform obese constitution by promoting shrinkage of general or topical fatty tissues, or to suppress or prevent obesity, a cosmetic composition comprising the same, a food or drink composition comprising the same, reducing body weight by a lipolitic function thereof and a drug comprising the same.

## Claims

1. Use of a compound represented by the following formula (1)

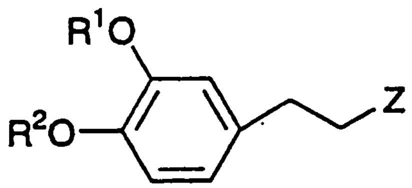 (1)

wherein Z is a group represented by the formula (2) or (3),

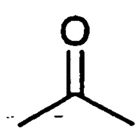 (2) 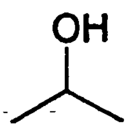 (3)

and either of $R^1$ and $R^2$ is a hydrogen atom and the other is a hydrogen atom or a glucose residue, as an active ingredient in the manufacture of a medicament for promoting lipolysis.

2. Use according to claim 1, wherein the active ingredient is 4-(3',4'-dihydroxyphenyl)-butane-2-one.

3. Cosmetic use of a compound according to claim 1 or 2 for reducing body weight.

4. Use according to claim 3, wherein the compound for promoting lipolysis is a cosmetic composition for reducing body weight.

5. Use according to claim 1 or 2, wherein the compound for promoting lipolysis is an anti-obesity drug.

6. Use according to claim 1 or 2, wherein the compound for promoting lipolysis is a remedy for hyperlipidemia.

7. Use according to claim 1 or 2, wherein the compound for promoting lipolysis is a remedy for fatty liver.

8. Food or drink composition, comprising a compound represented by the following formula (1)

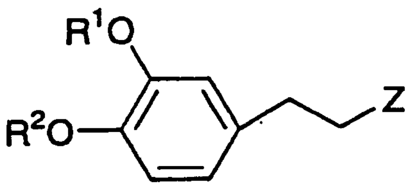 (1)

wherein Z is a group represented by the formula (2) or (3),

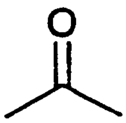 (2) 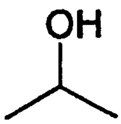 (3)

and either of $R^1$ and $R^2$ is a hydrogen atom and the other is a hydrogen atom or a glucose residue, as an active ingredient for promoting lipolysis.

**Patentansprüche**

1. Verwendung einer Verbindung, dargestellt durch die folgende Formel (1)

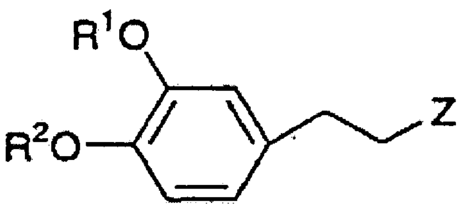 (1)

worin Z eine Gruppe ist, dargestellt durch die Formel (2) oder (3)

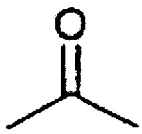 (2) 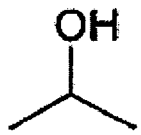 (3)

und eines von $R^1$ und $R^2$ ein Wasserstoffatom ist und das andere ein Wasserstoffatom oder ein Glucoserest ist, als ein aktiver Inhaltsstoff bei der Herstellung eines Medikamentes zum Fördern von Lipolyse.

2. Verwendung gemäß Anspruch 1, wobei der aktive Inhaltsstoff 4-(3',4'-Dihydroxyphenyl)-butan-2-on ist.

3. Kosmetische Verwendung einer Verbindung gemäß Anspruch 1 oder 2 zum Reduzieren von Körpergewicht.

4. Verwendung gemäß Anspruch 3, wobei die Verbindung zum Fördern der Lipolyse eine kosmetische Zusammensetzung zum Reduzieren von Körpergewicht ist.

5. Verwendung gemäß Anspruch 1 oder 2, wobei die Verbindung zum Fördern von Lipolyse ein Antiadipositas-Wirkstoff ist.

6. Verwendung gemäß Anspruch 1 oder 2, wobei die Verbindung zum Fördern von Lipolyse ein Arzneimittel für Hyperlipämie ist.

7. Verwendung gemäß Anspruch 1 oder 2, wobei die Verbindung zum Fördern von Lipolyse ein Arzneimittel für Fettleber ist.

8. Nahrungsmittel- oder Getränkezusammensetzung, umfassend eine Verbindung, dargestellt durch die folgende Formel (1)

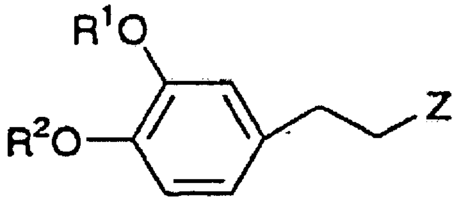 (1)

worin Z eine Gruppe ist, dargestellt durch die Formel (2) oder (3)

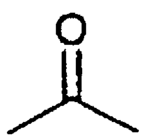 (2) 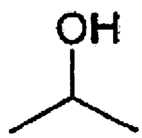 (3)

und eines von $R^1$ und $R^2$ ein Wasserstoffatom ist und das andere ein Wasserstoffatom oder ein Glucoserest ist, als einen aktiven Inhaltsstoff zum Fördern von Lipolyse.

## Revendications

1. Utilisation d'un composé représenté par la formule (1) ci-dessous:

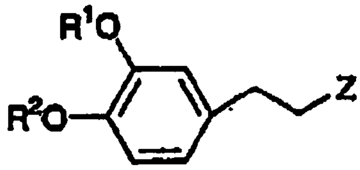 (1)

dans laquelle Z est un groupe représenté par la formule (2) ou (3)

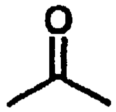 (2) 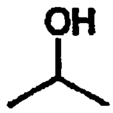 (3)

et l'un des $R^1$ et $R^2$ est un atome d'hydrogène et l'autre est un atome d'hydrogène ou un résidu de glucose, comme ingrédient actif dans la fabrication d'un médicament destiné à favoriser la lipolyse.

**2.** Utilisation selon la revendication 1, dans laquelle l'ingrédient actif est la 4-(3',4'-dihydroxyphényl)butane-2-one.

**3.** Utilisation cosmétique d'un composé selon la revendication 1 ou 2 pour réduire la masse corporelle.

**4.** Utilisation selon la revendication 3, dans laquelle le composé destiné à favoriser la lipolyse est une composition cosmétique pour réduire la masse corporelle.

**5.** Utilisation selon la revendication 1 ou 2, dans laquelle le composé destiné à favoriser la lipolyse est un médicament antiobésité.

**6.** Utilisation selon la revendication 1 ou 2, dans laquelle le composé destiné à favoriser la lipolyse est un remède pour l'hyperlipidémie.

**7.** Utilisation selon la revendication 1 ou 2, dans laquelle le composé destiné à favoriser la lipolyse est un remède pour la surcharge graisseuse des cellules hépatiques.

**8.** Composition alimentaire ou boisson, comprenant un composé représenté par la formule (1) ci-dessous:

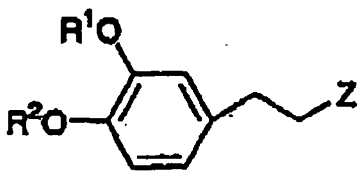 (1)

dans laquelle Z est un groupe représenté par la formule (2) ou (3)

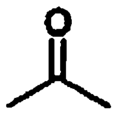 (2) 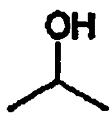 (3)

et l'un des $R^1$ et $R^2$ est un atome d'hydrogène et l'autre est un atome d'hydrogène ou un résidu de glucose, comme ingrédient actif destiné à favoriser la lipolyse.

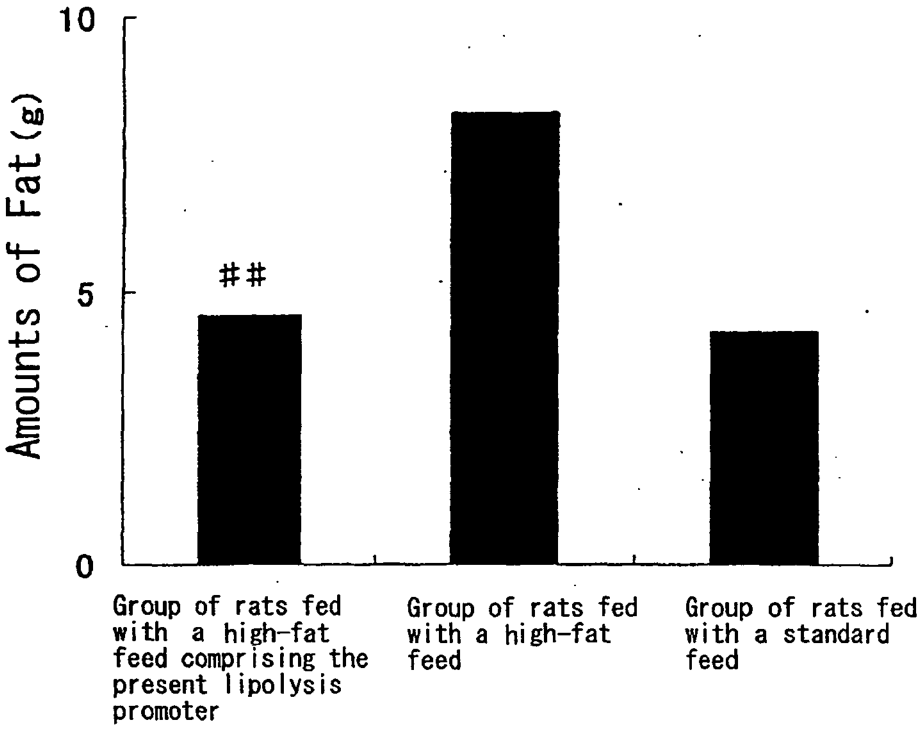
10
5
0
Amounts of Fat (g)
##
Group of rats fed with a high-fat feed comprising the present lipolysis promoter
Group of rats fed with a high-fat feed
Group of rats fed with a standard feed

Fig. 1

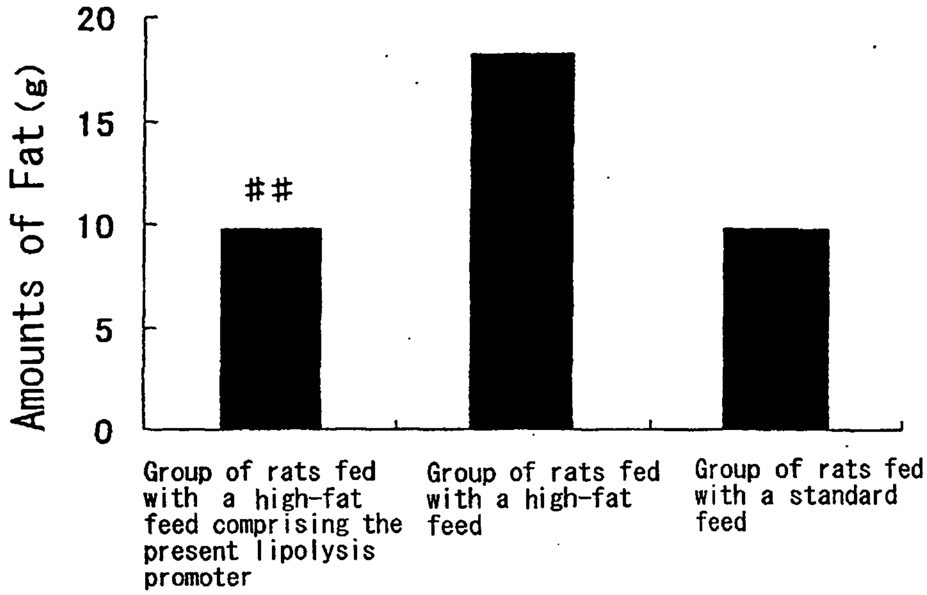
Amounts of Fat (g)
20
15
10
5
0
##
Group of rats fed with a high-fat feed comprising the present lipolysis promoter
Group of rats fed with a high-fat feed
Group of rats fed with a standard feed

Fig. 2

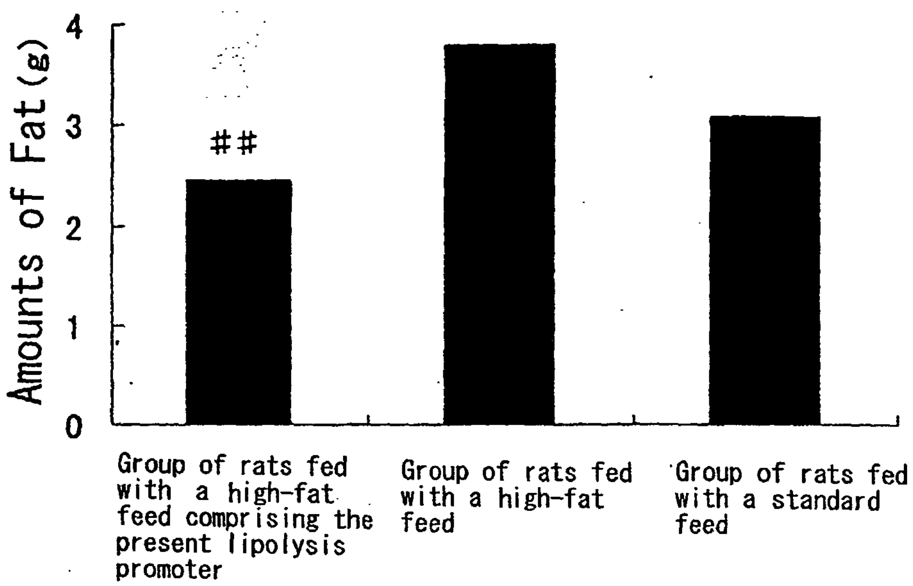
Amounts of Fat (g)
4
3
2
1
0
##
Group of rats fed with a high-fat feed comprising the present lipolysis promoter
Group of rats fed with a high-fat feed
Group of rats fed with a standard feed

Fig. 3

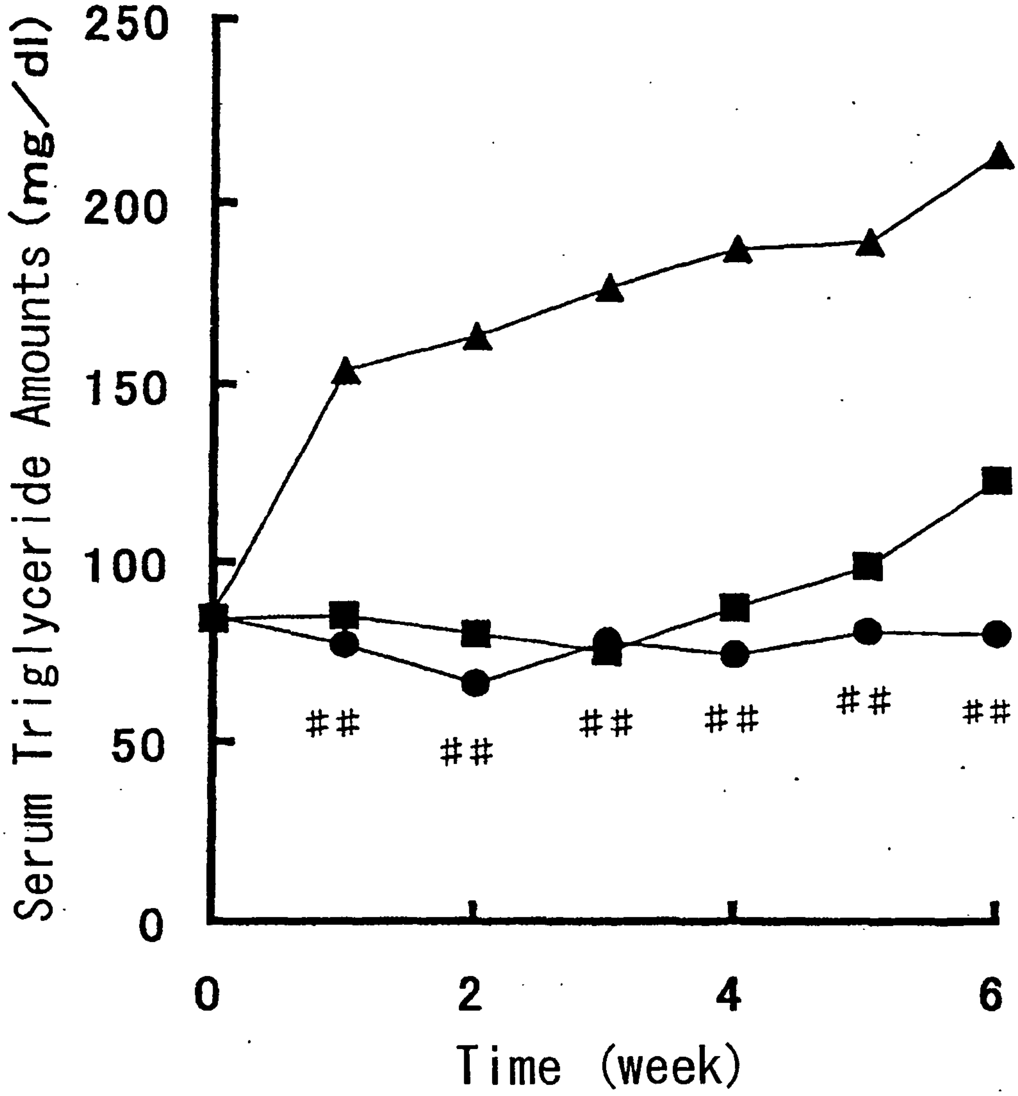
Serum Triglyceride Amounts (mg/dl)
250
200
150
100
50
0
##
##
##
##
##
##
0
2
4
6
Time (week)

Fig. 4